# EUROPEAN PATENT SPECIFICATION

(11) **EP 2 157 185 B1**
(45) Date of publication and mention of the grant of the patent: **14.05.2014**
(21) Application number: 08752455.9
(22) Date of filing: 08.05.2008
(51) Int. Cl.: C07C 51/43, C12P 7/46

(54) **Process for production of succinic acid, and process for production of ammonium succinate solution**
Verfahren zur Herstellung von Bernsteinsäure und Verfahren zur Herstellung einer Lösung des Ammoniumsalzes der Bernsteinsäure
Procédé de production d'acide succinique et procédé de production d'une solution de succinate d'ammonium

(30) Priority: 18.05.2007 JP 2007132917
(43) Date of publication of application: 24.02.2010
(73) Proprietor: Ajinomoto Co., Inc., Tokyo 104-8315 (JP)
(72) Inventor: NISHI, Kiyohiko, Saga-shi Saga 840-2193 (JP); KANEKO, Hiroshi, Saga-shi Saga 840-2193 (JP); TASAKI, Hirofumi, Saga-shi Saga 840-2193 (JP); KOGA, Masaki, Saga-shi Saga 840-2193 (JP); TANEGAWA, Testuo, Saga-shi Saga 840-2193 (JP); FURUYA, Seisaku, Saga-shi Saga 840-2193 (JP); FUJIWARA, Kenji, Saga-shi Saga 840-2193 (JP); SATO, Takeru, Saga-shi Saga 840-2193 (JP)
(74) Representative: Fischer, Heinrich
(86) International application number: PCT/JP2008/058569
(87) International publication number: WO 2008/143015

(56) References cited:
- EP-A1- 0 405 707
- EP-A1- 1 686 183
- EP-A2- 0 249 773
- WO-A1-99/09196
- JP-A- 03 030 685
- JP-A- 62 294 090
- JP-A- 2001 514 900
- JP-A- 2004 196 768
- JP-A- 2005 295 998

## Description

### Technical Field

This invention relates to a method of manufacturing succinic acid and ammonium succinate solutions derived from fermentation.

### Background Art

Succinic acid and its derivatives are widely used as raw materials for foods, perfumes and cosmetics, and medicines. Furthermore, succinic acid is recently noted its use for monomers of biodegradable plastics. Heretofore, succinic acid to be used for these applications has been produced by a petrochemical method. On the other hand, the production of succinic acid by fermentation is being investigated using carbohydrates of plant origin as raw materials in recent years.

In the case of producing succinic acid by the fermentation method, culture pH is, in general, needed to keep around neutral, so that microorganisms exhibit necessary and sufficient activities. As the neutralizing agent, calcium hydroxide, sodium hydroxide, magnesium hydroxide, ammonia, calcium carbonate, sodium carbonate, magnesium carbonate and ammonium carbonate, etc. are used. To use these brings the accumulation of corresponding succinic acid salts in the culture solution.

Among succinic acid salts, it is known that calcium succinate has a low solubility, and deposits as crystals in the culture solution (Patent Document 1). Usually, the culture solution contains a mixture of raw materials for fermentation, such as sugar, various additives added to culture medium as nutrients for microorganisms, and by-products produced by metabolism of microorganism, etc. As to calcium succinate, because it is possible to remove from the culture solution by separating deposited crystals from the mixture, purification processes of succinic acid can be rendered simple. Furthermore, since operations for deposition of succinate crystals, such as concentration, are not necessary, the deposition of calcium salt is advantageous in view of energy consumption.

A typical method of separating succinic acid from succinate salts is to lower pH into the region of generating free succinic acid by adding an acid, such as sulfuric acid. For calcium succinate, it is known a method of obtaining succinic acid by using sulfuric acid (Patent Document 2). However, this method has a difficulty in the deposition of calcium sulfate in quantity as a by-product.

As a method of separating succinic acid from succinates without acid, electrodialysis was proposed (Patent Document 3). Since bases which were separated from succinic acid during purification processes are reused as neutralizing agent in fermentation, by-products are not produced. However, the application of this method is limited to the succinic acid salts with monovalent bases, such as sodium succinate and ammonium succinate. For calcium salt, this method is unsuitable due to the depositon of scale component on the membrane.

By the aforementioned reasons, studies of using calcium compounds as a neutralizing agent have not been made in recent years.

On the other hand, in the method obtaining succinic acid from ammonium succinate by using sulfuric acid, ammonium sulfate is produced as a by-product. A method of no generating by-product salts finally was proposed by pyrolyzing the ammonium sulfate produced as a by-product at 300°C to produce ammonium hydrogen sulfate and ammonia, and reusing them in the process (Patent Document 4).

In addition, a method of using acetic acid was also proposed for obtaining succinic acid from ammonium succinate (Patent Document 5). By-product salts are not generated by pyrolyzing the ammonium sulfate produced as a by-product to produce acetic acid and ammonia, and reusing them in the process. As mentioned above, ammonium succinate is preferred as a starting material in the purification process of succinic acid.

Ammonium succinate can be accumulated in the culture solution by using ammonia on the like as a neutralizing agent in the fermentation process. However, it is disclosed that ammonia is not favorable as the neutralizing agent for producing succinic acid efficiently, and in view of productivity and yield in fermentation, magnesium compounds are effective (Patent Document 6).

Various purification methods are applicable to the magnesium salt which is in a favorable form in the fermentation process, and in view of no generating by-product salts by using the method disclosed in Patent Documents 4, 5, the salt substitution method was proposed as a method of converting into the ammonium salt which is a more favorable form (Patent Document 7). The magnesium compound produced in the salt substitution step is reused as a neutralizing agent in the fermentation process. Magnesium succinate which is produced in the fermentation process is dissolved in the culture solution, and usually is not deposited as crystals. Therefore, the salt substitution step is carried out by introducing ammonium carbonate or the like into the culture solution. As a result, the mixture in the culture solution other than magnesium succinate is carried into the ammonium succinate solution which is the starting material in the purification process. Therefore, there is a problem that a step for removing the complicated mixture is necessary before or after the salt substitution step.
Patent Document 1 : JP62-294090A
Patent Document 2 : JP3-30685A
Patent Document 3 : JP2-283289A
Patent Document 4 : JP2001-514900A
Patent Document 5 : JP2004-196768A
Patent Document 6 : WO2005/026349A
Patent Document 7 : JP2005-295998A

An object of the invention is to provide a method of manufacturing ammonium succinate solution efficiently, for the manufacture of succinic acid derived from fermentation.

### Disclosure of Invention

The inventors succeeded to produce calcium succinate efficiently by adding calcium compound, which is a neutralizing agent, in the fermentation process. Furthermore, they found that ammonium succinate solution can be manufactured with high purity by isolating calcium succinate, and then conducting salt substitution with ammonium compound, to complete the invention. Thus, the present invention includes the following processes:
1. A method of manufacturing an ammonium succinate solution with a high purity from fermentation comprising;
   (1) crystallization fermentation step wherein calcium succinate trihydrate is produced by microorganism;
   (2) transition step wherein calcium succinate trihydrate is converted to calcium succinate monohydrate by transition crystallization;
   (3) crystal separation step wherein calcium succinate monohydrate is separated;
   (4) salt substitution step wherein the calcium succinate monohydrate separated is made into ammonium succinate solution;
   (5) solid-liquid separation step wherein calcium carbonate is removed from the ammonium succinate solution.
2. The method of manufacturing the ammonium succinate solution as set forth in item 1 wherein calcium compound used in the fermentation is one or more selected from the group consisting of calcium hydroxide, calcium oxide, calcium hydrogen carbonate, and calcium carbonate.
3. The method of manufacturing the ammonium succinate solution as set forth in item 1 wherein reaction to convert calcium succinate trihydrate to calcium succinate monohydrate is carried out at 80°C or more.
4. The method of manufacturing the ammonium succinate solution as set forth in item 1 wherein the salt substitution step to substitute ammonium succinate for calcium succinate monohydrate is carried out by ammonium carbonate or ammonia and carbon dioxide gas.
5. The method of manufacturing the ammonium succinate solution as set forth in items 1-4 wherein succinic acid in the salt substitution step has a concentration of 5% by weight or more.
6. A method of manufacturing succinic acid comprising producing an ammonium succinate solution according to the method as set forth in either one of items 1-5, and obtaining succinic acid from said ammonium succinate solution.

From the ammonium succinate solution, succinic acid can be manufactured by a known method (Patent Document 4, 5, etc.). In addition, calcium carbonate produced by the salt substitution can be reused as a fermentation neutralizing agent. Alternatively, it is also possible to neutralize by calcium hydroxide which is produced by calcining calcium carbonate by a known method (Non-Patent Document 1) and hydrating the produced calcium oxide. Further, carbon dioxide generated by the calcination can be used effectively in the fermentation and salt substitution steps.

Non-Patent Document 1; Ed. by Sekko Sekkai Gakkai, "Sekko Sekkai Handbook (Gypsum • Line Handbook)", pp126-144, Gihodo Shuppan, 1972.

According to the invention, ammonium succinate solution is possible to be manufactured efficiently without complicated processes, and from the ammonium succinate solution, succinic acid is manufactured by a known method, and only reusable by-products are produced.

### Brief Description of The Drawings

[Figure 1] Photographs illustrating a comparison of crystal forms between calcium succinate trihydrate (A) obtained by using a high purity raw material (reagent level) and calcium succinate trihydrate (B) accumulated in a fermentation solution.
[Figure 2] Photographs illustrating a comparison of crystal forms between calcium succinate trihydrate (A) accumulated in a fermentation solution and calcium succinate monohydrate (B) obtained by the transition of (A).

### Best Mode for Carrying Out The Invention

The present invention is, as mentioned above, an invention constituted by 5 step processes, and hereafter, their contents are described in order.

### (1) Fermentation Step

The present invention starts from the production of succinic acid in a culture solution produced by fermentation. To the manufacture of succinic acid by culture, a known method from long ago is applied wherein a strain capable of producing succinic acid is cultured while neutralized with calcium compound to produce succinic acid. Although microorganisms applicable to the invention are not particularly limited, preferred are aerobic bacteria or facultative anaerobic bacteria, specifically coryneform bacterium, Bacillus genus bacterium, Rhizobium genus bacterium or Escherichia genus bacterium, and, among them, coryneform bacterium is more preferred. As the coryneform bacterium, microorganisms belonging to Corynebacterium genus, microorganisms belonging to Brevibacterium genus or microorganisms belonging to Arthrobacter genus can be listed, and preferred microorganisms are belonging to Corynebacterium genus or Brevibacterium genus. More preferred are microorganisms belonging to Corynebacterium glutamicum, Brevibacterium flavum, Brevibacterium ammoniagenes or Brevibacterium lactofermentum.

Particularly preferred examples of the above microorganisms are Brevibacterium flavum MJ-233 (FERM BP-1497), MJ-233 AB-41 (FERM BP-1498) which is a mutant of the MJ-233, Brevibacterium ammoniagenes ATCC 6872, Corynebacterium glutamicum ATCC 31831, ATCC 13032 and Brevibacterium lactofermentum ATCC 13869 and the like. Since Brevibacterium flavum is sometimes classified in Corynebacterium glutamicum now (Non-Patent Document 2), in the invention, Brevibacterium flavum MJ-233 strain and its mutant MJ-233 AB-41 strain can be called Corynebacterium glutamicum MJ-233 strain and MJ-233 AB-41 strain. Non-Patent Document 2: Lielbl, W., Ehrmann, m., Ludwig, W. and Schleifer, K. H., International Journal of Systematic Bacteriology, 1991, vol. 41, p255-260.

Strains to be used are, of course, the above strains, and may be strains having improved productivity of succinic acid obtained from the above strains as a parent strain by mutation or gene manipulation. For example, a mutant, Corynebacterium glutamicum MJ 233/ Δldh which lacks lactate dehydrogenase can be used. The mutant can be structured by the method described in Patent Document 8. Furthermore, the fermentation process may be divided into a bacterial cell culture step and a succinic acid fermentation step for the purpose of improving productivity.

Patent Document 8; WO 2005/021770

### a) Bacterial Cell Culture Step

On culturing the above microorganism, it is desirable to conduct under aerobic atmosphere with aeration and stirring.

Hereupon, "under aerobic atmosphere" means the conditions where bacterial cells are multiplied with consuming oxygen. Although dissolved oxygen concentration is not particularly limited, for efficient multiplication, it is 4% or more, more preferably 20% or more, as dissolved oxygen saturation.

As the culture medium for cultivation, usual nutrient medium can be used containing carbon source, nitrogen source, inorganic salts and optional other organic minor nutrients, such as amino acids and vitamins. Either synthetic medium or natural medium is usable. The carbon source and nitrogen source used in the culture medium may be any one utilizable by the strain to be cultured.

As the carbon source, sugars, such as glucose, glycerol, fructose, sucrose, maltose, mannose, galactose, starch hydrolyzate, and molasses, and moreover, organic acids, such as acetic acid and citric acid may be used singly or in combination with other carbon sources.

As the nitrogen sources, ammonia, ammonium salts, such as ammonium sulfate, ammonium carbonate, ammonium chloride, ammonium phosphates and ammonium acetate, and nitrates are usable.

As the organic minor nutrients, amino acids, vitamins, fatty acids, nucleic acids, and moreover, materials containing these, such as peptone, casamino acids, yeast extracts and soybean protein hydrolyzates are used. When an auxotroph mutant is used requiring amino acids, or the like for its metabolism or growth, it is necessary to supply the nutrient to be required.

As the inorganic salts, phosphates, magnesium salts, calcium salts, iron salts, manganese salts and the like are used.

Cultivation form is not particularly limited, and however, in order to obtain bacterial cells in a higher concentration, fed batch culture and continuous culture are preferable where substrate is sequentially added, and bacterial cell yield can be raised.

Culture temperature and pH are not particularly limited, so far as being possible to multiply. Usually, temperature is in the range of 20 to 40°C, and pH is in the range of 6.0 to 8.0.

Cultivation can be carried out until desired cell amount is obtained. Preferable amount is in the range of 30 to 120g/l as dry cell weight.

### b) Calcium Succinate Trihydrate Crystallization Fermentation Step

Succinic acid fermentation is carried out by reacting the bacterial cells, which have been cultured above, with carbon source in an anaerobic atmosphere, and neutralizing produced succinic acid with a calcium compound. By reacting under suitable conditions for cultured bacterial cells, succinic acid can be produced and accumulated in the culture medium with depositing calcium succinate trihydrate.

The "In an anaerobic atmosphere" means that dissolved oxygen concentration is 0.1 ppm or less, preferably 0.01 ppm or less. In order to make "in an anaerobic atmosphere", oxygen supply is restricted. As the method therefor, to reduce aeration, to render stirring mild, to blow gas of which oxygen partial pressure has been reduced by mixing inert gas, such as nitrogen gas or carbon dioxide gas, or the like can be employed. The anaerobic atmosphere can be made complete anaerobic atmosphere where oxygen is entirely removed. As the method therefor, to stop aeration, to blow only inert gas, or the like can be employed.

The quantity of the bacterial cells cultured or above to be used for the reaction is not particularly limited, and however, is usually 1 to 120g/l, preferably 10 to 80g/l. A bacterial cell culture solution can be used as is in a desired volume. Alternatively, mixed materials in the culture solution may be removed by an operation, such as centrifuging, prior to the use for reaction.

The carbon source to be used for succinic acid fermentation is not particularly limited so far as it can be digested by the microorganism to produce succinic acid, and carbon sources similar to bacterial cell culture can be used.

The method for addition of carbon source is also not particularly limited, and for example, the total amount of carbon source necessary for the desired amount of succinic acid has been added previously at the start of reaction. Alternatively, carbon source may be added successively so as to conform with the consumption of the carbon source in accordance with reaction proceeding. Furthermore, the total amount of carbon source may be added successively.

The calcium compounds usable for neutralization of succinic acid are calcium carbonate (CaCO₃)), calcium oxide (CaO), calcium hydroxide (Ca(OH)₂), calcium hydrogen carbonate (Ca(HCO₃)₂) and the like, and although not particularly limited, use of calcium hydroxide is preferable in view that pH can be easily controlled. In addition, two kinds or more of these can be combined.

As to the method of adding the calcium compound, it is not particularly limited within the range of capable of controlling pH to an appropriate value. For example, by rendering in a slurry state, the calcium compound can be added in accordance with pH. It may be add in the form of powder.

The reaction temperature, pH are not particularly limited within the range capable of producing succinic acid, and in general, the temperature is in the range of 20 to 40°C, and the pH is in the range of 4.0 to 8.0.

As to the culture medium to be used for reaction, it is not particularly limited within the range of not inhibiting succinic acid producing reaction, and for example, it may contain only carbon source. In addition, additives other than carbon source may be added similar to the above bacterial cell culture step. For example, phosphoric acid compounds, such as or the phosphoric acid, may be added as reaction accelerator. The addition method of the additives other than carbon source is not particularly limited, and for example, the total amount may be added on starting the reaction. Alternatively, it may be added successively during the reaction proceeds.

Moreover, it is preferred to incorporate carbonate ion, bicarbonate ion or carbon dioxide gas in the culture medium. The supply method is not particularly limited, and for example, carbon dioxide is aerated. A preferred concentration of carbon dioxide gas to be aerated is 5% or more. Meanwhile, when calcium carbonate is used as neutralizing agent, carbonate ion and bicarbonate ion can also be supplied.

The form of the fermenter to be used for the reaction is not particularly limited, and for example, a mechanical stirring type fermenter commonly used for amino acid fermentation and the like can be used. A bubble tower type culture vessel (e. g. Patent Document 9) can also be used.
Patent Document 9: JP11-092502A

With reaction proceeding, calcium succinate trihydrate crystals deposit. The reaction continues until a desired amount of calcium succinate is produced in the reaction solution. In general, the reaction continues until calcium succinate is accumulated in an amount of 30g/l or more, preferably 50g/l or more as the weight of succinic acid. The reaction time is usually 10 to 48 hours. The reaction may further continue with extracting deposited crystals out of the fermenter.

### (2) Transition Step

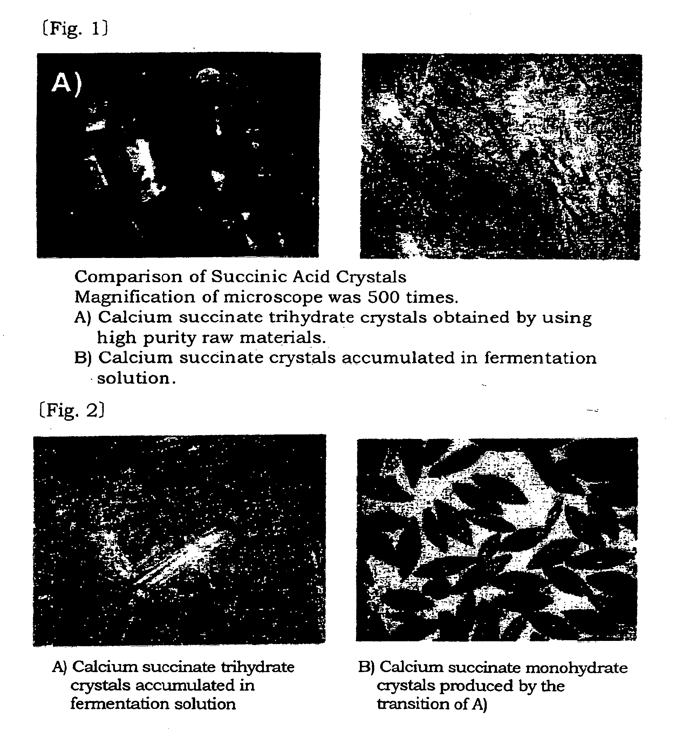

The calcium succinate trihydrate crystals which precipitates in the reaction solution can be collected by centrifugation or filtration. However, as shown in Figure 1, the calcium succinate trihydrate crystals produced by fermentation are considerably fine needle crystals compared with calcium succinate trihydrate crystals produced under high purity conditions, for example, calcium succinate trihydrate produced by reacting guaranteed grade succinic acid with calcium hydroxide manufactured by Wako Junyaku Co., Ltd. By transferring the crystals to calcium succinate monohydrate crystals, which are easier to separate, the separation of calcium succinate crystals is made easy from the reaction solution containing mixed materials, such as bacterial cells and reaction by-products.

The transition reaction is carried out by heating the reaction solution. The transition point from the trihydrate to the monohydrate is at 60°C or around there for pure reagents and higher than that and up to 80°C or around there for culture solution. Thereupon, optimum operation conditions are different according to the reaction solution composition, and in general, are conducted by heating to 80°C or more. The reaction time depends on temperature, and in the case of the reaction temperature of 80°C, it is usually for 2 to 24 hours. These conditions can be determined easily for a person skilled in the art in a suitable range by the observation of crystal state.

As shown in Figure 2, crystal form varies from needle shaped crystals (calcium succinate trihydrate) in the reaction solution to ellipsoid shaped crystals (calcium succinate monohydrate) by this transition step. The monohydrate is relatively stable, and when temperature lowers, it does not return to the trihydrate easily.

### (3)Crystal Separation Step

In this step, calcium succinate monohydrate crystals are separated, for example, around 60°C from the reaction solution which has been conducted the transition crystallization. The reaction solution contains bacterial cells, reaction by-products and the like in addition to calcium succinate monohydrate crystals. The method of separating calcium succinate monohydrate crystals from these is not particularly limited, but in view of efficient separation, separation utilizing the difference of specific gravity is preferred.

For separation, screw decanter, super decanter and the like are usable.

Separated calcium succinate monohydrate crystals are delivered to the next step. Prior to the delivery, the crystals may be made into slurry again for the purpose of the removal of bacterial cells and impurities, and washing. The liquid to be used for washing may be water. However, when solution of calcium succinate, preferably saturated solution, is used, loss of succinic acid can be prevented. By separating the slurry thus obtained, calcium succinate monohydrate crystals can be obtained again.

Since calcium succinate monohydrate is dissolved 10 to 20g/ℓ as succinic acid in the reaction solution, the dissolved portion moves to the side of mother liquor when separating. For the purpose of raising recovery of calcium succinate monohydrate crystals, for example, it is possible to deposit further crystals by concentrating the separated mother liquor. In addition, deposited crystal quantity may be raised by concentrating the reaction solution prior to the separation step. Alternatively, the mother liquor is returned to the fermentation step, and thereby, recovery of calcium succinate monohydrate crystals as a whole.

### (4) Salt Substitution Step

This step is the step of conducting the salt substitution of calcium succinate monohydrate to ammonium succinate and of producing and depositing calcium carbonate. By this reaction, succinic acid produced as calcium salt in the fermentation step can be converted to ammonium salt solution.

By mixing calcium succinate monohydrate and ammonium carbonate to react them, the salt substitution is conducted. Alternatively, the salt substitution reaction may be carried out by adding ammonia and carbon dioxide gas. These conditions can be varied appropriately for a person skilled in the art.

A preferred succinic acid concentration in the reaction solution is in the range of not depositing crystals of ammonium succinate produced by the salt substitution, and in general, is 5 to 40% by weight of succinic acid. The succinic acid concentration may be adjusted easily by controlling the volume of water to be added to calcium succinate monohydrate crystals upon the salt substitution reaction.

In the case of using ammonium carbonate for the salt substitution, in general, the carbonate is added in an amount of 0.5 to 5 times, preferably 0.5 to 2 times moles that of the calcium in the reaction solution. The "calcium in the reaction solution" referred to herein means the total amount of the calcium contained in calcium succinate and calcium compounds. The calcium in the reaction solution is precipitated and deposited as calcium carbonate by reacting with carbonate ion.

In the case that the reaction is carried out by ammonia and carbon dioxide gas, the carbon dioxide gas is added in a volume of 0.5 to 5 times, preferably 0.5 to 2 times moles that of the calcium in the reaction solution. The ammonia to be added may be in either form of aqueous ammonia or ammonia gas. The addition volume of ammonia is arranged so that the pH of the reaction solution becomes into the range capable of precipitating calcium carbonate. The pH range is usually 5.0 to 9.0, preferably 6.0 to 8.0.

It is preferred to conduct the reaction with stirring. The reaction time is not particularly regulated, and the reaction is, in general, finished for 0.5 to 5 hours.

### (5) Solid-Liquid Separation Step for Removal of Calcium Carbonate

By this step, calcium carbonate can be removed from the slurry mixture of ammonium succinate solution and calcium carbonate precipitates, which was produced in the salt substitution step, to obtain an ammonium succinate solution.

The separation of calcium carbonate may be carried out by either filtration or specific gravity separation.

According to this step, a high purity ammonium succinate solution can be obtained where the total of succinic acid and ammonia occupies 90% or more of the total solid component.

The separated calcium carbonate can be reused in the fermentation process. Alternatively, calcium hydroxide, which is a preferable neutralizing agent for the fermentation process, can be produced by known calcination and hydration methods.

Moreover, the carbon dioxide gas generated by the calcination can be recovered and reused in the fermentation step or the salt substitution step.

Using the ammonium succinate solution obtained in the invention, succinic acid can be produced by a known method. The method of producing succinic acid is not particularly limited, and for example, a method of using acetic acid (Patent Document 5) can be employed.

### Examples

The invention will be explained in detail hereinafter with reference to Examples.

### [Example 1]

Hereafter, illustrates as to the crystallization fermentation step for manufacturing calcium succinate trihydrate by using the strain Corynebacterium glutamicum MJ233/Δldh and neutralizing with calcium hydroxide, the transition step for producing calcium succinate monohydrate by heating the culture solution, the crystal separation step for separating crystals from the reaction solution, the salt substitution step for substituting from the calcium salt to the ammonium salt, and the solid-liquid separation step for obtaining calcium carbonate deposited from the ammonium salt solution.

### <Fermentation Step>

### <Seed Culture>

A 100ml culture medium containing 30g/l glucose, 0.4g/l magnesium sulfate 7 hydrates, 1.0g/l potassium dihydrogen phosphate, 12g/l urea, 0.5g/l DL-methionine, 10mg/l ferrous sulfate 7 hydrates, 10mg/l manganese sulfate 5 hydrates, 0.3mg/l D-biotin, 0.2mg/l thiamin hydrochloride, 28.6ml/l soybean protein hydrolyzate (containing 35g/l total nitrogen) and 10g/l yeast extract, was placed in a 50ml Sakaguchi's flask, and sterilized with heating at 120 °C for 20 minutes. After cooling to room temperature, the strain Corynebacterium glutamicum MJ233/Δ ldh was seeded, and cultured with shaking at 31.5°C for 22 hours.

### <Bacterial Cell Culture>

1500ml of a culture medium containing 20g/l glucose, 2.0g/l magnesium chloride 7 hydrates, 2.0g/l orthophosphoric acid, 1.0g/l citric acid monohydrate, 14.3ml/l soybean protein hydrolyzate (containing 35g/l total nitrogen), 0.8g/l ammonium chloride, 1.0g/l DL-methionine, 200mg/l manganese sulfate 5 hydrates, 3mg/l D-biotin, 1mg/l thiamin hydrochloride and 0.25ml/l anti-foaming agent was prepared, and sterilized at 120°C for 20 minutes. After cooling to room temperature, the culture medium was placed in a 51 reaction vessel, and 9ml of the above seed culture solution was added, and kept at 31.5°C. Stirring culture was conducted with aerating at 1.51/min and adjusting pH to 6.6 by ammonia gas. After 8.0 hours of the culture, previously sterilized glucose solution (500g/l) was supplied continuously at a rate of 25ml/hour until the culture was finished. When 340ml of the glucose solution was introduced, the culture was finished to obtain a bacterial cell culture solution containing about 50g/l bacterial cell.

### <Succinic Acid Crystallization Fermentation>

A culture medium containing 100g/l glucose, 2.0g/l orthophosphoric acid and 0.05ml/l anti-foaming agent (the above concentrations are after seeding the bacterial cell culture solution.) was prepared, and placed in a 51 reaction vessel. After sterilizing at 120°C for 20 minutes, the culture medium was kept at 34°C, and 840ml of the above bacterial cell culture solution was added. The total volume was 2.11, and the stirring culture was carried out. In the stirring culture, a mixed gas having an air : nitrogen gas : carbon dioxide gas ratio of 10 : 70 : 20 was aerated at 210ml/min., and pH was kept neutral region of pH6.6 by adding calcium hydroxide slurry (260g/l).

The reaction was continued for 24 hours. As a result, introduced glucose was completely consumed, and 2.89kg (2.651) of the reaction solution where 57.0g/l succinic acid was accumulated was obtained. Conversion yield of glucose to succinic acid was 72%. The succinic acid concentration of supernatant of the reaction solution was 13.3g/l, and therefore, it was calculated that 77% of succinic acid in the reaction solution exists in a form of calcium salt. The culture was conducted similarly using 4 vessels, 589g succinic acid was obtained as the calcium salt from 11.17kg (10.751) of the reaction solution in the total

### <Transition Step>

9.70kg of the above reaction solution was placed in a stainless steal vessel having a total volume of 301, and heated to 80°C in a water bath. The temperature was kept for about 18 hours with stirring. Through the operations, change of crystal form was observed, and as a result of x-ray diffraction, it was found that calcium succinate trihydrate crystals were converted to calcium succinate monohydrate crystals. Before and after the transition reaction, evaporation of water was observed by heating, but the quantity of succinic acid was not varied. 9.28kg of the reaction solution was obtained, and 571g succinic acid was obtained. The succinic acid oncentration of the supernatant of the reaction solution was 15.4g/l, and the solubility of succinic acid was not greatly varied before and after the transition reaction.

### <Crystal Separation Step>

After cooling 9.28kg of the reaction solution, wherein transition with heating had been achieved, to about 60 °C, solid-liquid separation was carried out by a screw decanter. The decanter used was manufactured by Tomoe Industries Co., Ltd., and set at 1334rpm for the inner cylinder and 3000rpm for the outer cylinder. The reaction solution was introduced at a flow rate of 301/hour. The separation was finished after about 20 minutes, and adhered portions on the cylinders were combined with the separated cake. Separated mother liquor was 8.07kg and the cake was 0.99kg. The analyzed results before and after the separation are shown in table 1. 67.2% of succinic acid in the reaction solution was recovered. 38.4% of the separated cake was succinic acid. The cake was evaporated to dryness, and weight was measured. As a result, it was found that succinic acid and calcium occupied 81.9% of the total solid matter. It was confirmed that 70% or more of the introduced bacterial cells were removed on the side of the separated mother liquor.

**[Table 1]**

| | Before Separation | Separated Cake | Separated Mother Liquor |
|---|---|---|---|
| Weight (kg) | 9.28 | 0.99 | 8.07 |
| Succinic Acid (wt%) | 6.16 | 38.4 | 1.22 |
| Acetic Acid (wt%) | 0.73 | 0.72 | 0.75 |
| Ca Conc. (wt%) | 2.73 | 13.96 | 0.99 |
| Succinic Acid (g) | 572 | 380 | 98 |
| Ca/Succinic Acid (mol/mol) | 1.28 | 1.05 | 2.37 |
| Total Solid Matter (g) | 1239 | 634 | 468 |
| Succinic Acid/Total Solid Matter (wt%) | 46.1 | 60.1 | 21.0 |
| Bacterial Cell Quantity (g) | 89.9 | 24.8 | 62.9 |

### <Salt Substitution Step>

630g (containing 242g succinic acid) of the cake recovered in the above separation step was used for the salt substitution step. The cake was placed in a 5,000ml beaker, and 1.58kg water was added. The cake was suspended by stirring at ordinary temperature to form a slurry solution. Subsequently, 175g ammonium carbonate (guaranteed grade, Wako Junyaku Co., Ltd., which was 0.8 time mole of the calcium in the slurry, was introduced, and allowed to react with stirring at ordinary temperature for 2 hours. 2.24kg of the salt substitution reaction solution was obtained which was in a slurry state.

### <Solid-Liquid Separation Step>

2.24kg of the slurry solution obtained in the above salt substitution reaction was separated into the filtrate and precipitates by a Nutsche funnel filtration. Finally, 1.82kg of the solution and 0.42kg of the precipitates were obtained. The molar ratio of the succinic acid in the solution was 1.92 to ammonia and 0.13 to calcium, respectively. It was found that 91% of calcium was removed as precipitates by the salt substitution reaction, and an ammonium succinate solution could be obtained. Calcium in the removed precipitates was 32.2% of the total solid matter, and succinic acid was less than 8.0%.

The analyzed results of the solution obtained in the salt substitution and solid-liquid separation step are shown in Table 2. The succinic acid occupied 72.7% of the total solid matter. It was found that the succinic acid and ammonia occupied 93.1% of the total solid matter, and a high purity ammonium succinate solution was obtained.

**[Table 2]**

| | Solution after Substitution • Separation |
|---|---|
| Weight (kg) | 1.82 |
| Succinic Acid (wt%) | 10.1 |
| Acetic Acid (wt%) | 0.23 |
| Ca Conc. (wt%) | 0.44 |
| NH₃ Conc. (wt%) | 2.83 |
| Ca/Succinic Acid (mol/mol) | 0.13 |
| NH₃/Succinic Acid (mol/mol) | 1.92 |
| Succinic Acid/Total Solid Matter (wt%) | 72.7 |
| Succinic acid + NH₃/Total Solid Matter (wt%) | 93.1 |

### [Example 2]

Influence of the quantity of ammonium carbonate in the salt substitution step was examined. To 80g calcium succinate monohydrate (Junsei Kagaku Co., Ltd., guaranteed grade), 188g water was added to form a slurry solution with a 20% by weight of succinic acid concentration. To this solution, ammonium carbonate was added with varying the quantity. After reacting for 2 hours with stirring, ammonium succinate solutions were obtained by centrifugation to remove precipitates.

The analyzed results of the obtained ammonium salt solutions are shown in Table 3. It indicates that 97.7% of calcium was removed from the solution by adding equimolar ammonium carbonate with the calcium in the slurry, and the succinic acid was recovered as an ammonium salt solution. It was found that calcium was removed at almost 100% by rendering ammonium carbonate slightly excess, i, e, 1.5 times the quantity of calcium, and succinic acid became ammonium salt completely.

**[Table 3]**

| Experiment No. | 1 | 2 | 3 | 4 |
|---|---|---|---|---|
| Ammonium Carbonate (mol/mol Charged Ca) | 1 | 1.5 | 2 | 4 |
| Reaction Solution pH | 7.29 | 7.81 | 8.26 | 8.23 |
| Succinic Acid (wt%) | 22.0 | 23.0 | 21.2 | 16.7 |
| NH₃ Conc. (wt%) | 5.6 | 6.5 | 6.8 | 11.4 |
| Ca Conc. (wt%) | 0.21 | 0.01 | 0.00 | 0.01 |
| NH₃/Succinic Acid (mol/mol) | 1.66 | 1.97 | 2.23 | 4.48 |
| Ca/Succinic Acid (mol/mol) | 0.03 | 0.01 | 0.00 | 0.02 |
| Ca Removal Rate (1-Solution Ca Quantity/Charged Ca Quantity) x 100 | 97.7 | 99.9 | 100.0 | 99.8 |

### [Example 3]

Using the calcium succinate monohydrate crystals obtained from the reaction solution similar to Example 1, experiments were carried out similar to Example 2 with varying the quantity of ammonium carbonate. To 50g calcium succinate crystals (succinic acid : 19.2g), 153g water was added, and suspended with stirring to form a 10wt. % succinic acid slurry. Ammonium carbonate was added, and allowed to react at ordinary temperature for 2 hours with stirring. After the reaction, precipitates were removed by centrifugation to obtain an ammonium succinate solution.

The analyzed results of the obtained ammonium salt solutions are shown in Table 4. It indicates that 92% or more of the calcium in the slurry can be removed as precipitates by adding ammonium carbonate in a quantity 0.5 time mole or more of the calcium in the slurry. It also indicates that 99% or more of the calcium in the slurry can be removed as precipitates by adding ammonium carbonate in a quantity 1.5 times mole or more.

**[Table 4]**

| Experiment No. | 1 | 2 | 3 | 4 |
|---|---|---|---|---|
| Ammonium Carbonate (mol/mol Charged Ca) | 0.5 | 0.9 | 1.5 | 1.8 |
| Reaction Solution pH | 7.31 | 7.50 | 8.40 | 8.40 |
| Succinic Acid (wt%) | 8.86 | 9.08 | 9.48 | 9.32 |
| NH₃ Conc. (wt%) | 1.47 | 2.51 | 3.73 | 4.61 |
| Ca Conc. (wt%) | 0.33 | 0.31 | 0.01 | 0.03 |
| NH₃/Succinic Acid (mol/mol) | 1.79 | 1.95 | 3.19 | 3.26 |
| Ca/Succinic Acid (mol/mol) | 0.17 | 0.10 | 0.00 | 0.01 |
| Ca Removal Rate (1-Solution Ca Quantity/Charged Ca Quantity) x 100 | 92.0 | 92.5 | 99.6 | 99.3 |

### [Example 4]

Experiments were carried out wherein salt substitution was conducted as to crystals obtained similar to Example 1 wherein the concentration of charging slurry in the salt substitution step was varied. The introduced quantity of ammonium carbonate was equimolar with the calcium in the slurry. After the salt substitution reaction, precipitates were removed by centrifugation to obtain an ammonium succinate solution.

The analyzed results of the obtained ammonium salt solutions are shown in Table 5. In all Experiments Nos. 1, 2, 3, it was indicated that 99% or more of the charged calcium can be removed as precipitates by conducting salt exchange. Moreover, in Experiment No. 3, the succinic acid concentration of the solution after substitution reached 28 wt.%, and it indicates that a high concentration ammonium succinate solution can be obtained by conducting salt exchange of calcium succinate without concentration.

**[Table 5]**

| Experiment No. | 1 | 2 | 3 |
|---|---|---|---|
| Succinic Acid in Slurry before Salt Substitution (wt%) | 25 | 30 | 35 |
| Ammonium Carbonate (mol/mol Charged Ca) | 1.0 | 1.0 | 1.0 |
| Reaction Solution pH | 8.21 | 7.94 | 8.19 |
| Succinic Acid (wt%) | 17.5 | 23.0 | 28.4 |
| NH₃ Conc. (wt%) | 8.1 | 9.6 | 10.5 |
| Ca Conc. (wt%) | 0.02 | 0.02 | 0.02 |
| NH₃/Succinic Acid (mol/mol) | 2.88 | 2.59 | 2.29 |
| Ca/Succinic Acid (mol/mol) | 0.0 | 0.0 | 0.0 |
| Ca Removal Rate (1-Solution Ca Quantity/Charged Ca Quantity) x 100 | 99.9 | 99.9 | 99.9 |

### [Example 5]

### <Collection of Succinic Acid Crystals>

Collection of succinic acid crystals was carried out from the ammonium succinate solution obtained in Example 1 by the acetic acid addition method (JP 2005-139137). 1.72kg of the ammonium succinate solution was concentrated by a rotary evaporator at 70°C under reduced pressure to obtain 400g of a 38.4 wt. % succinic acid solution. The concentrated solution was taken in a beaker, and 193g of acetic acid, which is 2.4 times mole of succinic acid, was added with stirring. Further, 120g of methanol, which was 20 wt. % against the reaction solution, was added to produce a slurry solution.

The slurry solution was filtered to obtain 113.6g crystals. The crystals contained 61.4% succinic acid and 8.7% ammonia. The molar ratio of succinic acid and ammonia was 0.97, and it was found that mono-ammonium succinate crystals were produced.

194g acetic acid was added to the crystals produced above, and allowed to react with stirring. The reacted slurry solution was filtered to obtain 58.2g crystals. The succinic acid content, acetic acid content and ammonia content of the crystals were 61.1%, 35.6% and 1.2%, respectively.

The crystals obtained above were washed with 5 times volume of water followed by filtering, and then, dried. The purity of succinic acid of the crystals thus obtained was 96.7%. The ratio by weight of ammonia and calcium to succinic acid were 0.03% and 0.04%, respectively, and it was confirmed that succinic acid crystals were produced.

### [Comparative Example 1]

As a comparison, neutralization with ammonia was carried out in the fermentation step. An ammonium succinate solution was obtained by removing bacterial cells from the reaction solution.

### <Fermentation Step>

Corynebacterium glutamicum MJ233/ Δldh strain was used. The bacterial cell culture and succinic acid fermentation were carried out similar to Example 1, wherein neutralization of the reaction solution was conducted using 120 g/l aqueous ammonia instead of calcium hydroxide.

In 24 hours culture, about 30g/l glucose remained, and therefore, the reaction was further continued up to 48 hours. As a result, glucose was consumed completely to obtain 2.34kg (2.181) of a culture solution containing 32.2g/l succinic acid. The yield of conversion from glucose to succinic acid was 33%.

### <Bacterial Cell Removal Step>

Bacterial cells were removed from the culture solution obtained in the above fermentation step by centrifugation to obtain an ammonium succinate solution. The succinic acid concentration was 3.17 wt. %, and the molar ratio of ammonia and succinic acid was 2.08. The solution was dried, and the rate of succinic acid occupied in the total solid matter was measured and found to be 44.2%. The rate of succinic acid + ammonia occupied in the total solid matter was 57.5%.

### [Comparative Example 2]

As a comparison, neutralization with magnesium was carried out. Bacterial cells were removed from the reaction solution to obtain a magnesium salt solution, and the salt substitution was conducted by ammonium carbonate to obtain an ammonium succinate solution.

### <Fermentation Step>

Corynebacterium glutamicum MJ233/ Δ ldh strain was used. The bacterial cell culture and succinic acid fermentation were carried out similar to Example 1 wherein neutralization of the reaction solution was conducted using 205g/l magnesium hydroxide slurry instead of calcium hydroxide.

In 24 hours culture, about 30g/l glucose remained, and therefore, the reaction was further continued up to 48 hours. Nevertheless, glucose still remained in a concentration of about 10g/l. 2.57kg (2.401) of a culture solution containing 41.4g/l of succinic acid was obtained. The yield of conversion from consumed glucose to succinic acid was 56%.

### <Bacterial Cell Removal Step>

Bacterial cells were removed from the culture solution obtained in the above fermentation step by centrifugation to obtain a magnesium succinate solution. The succinic acid concentration of the solution was 4.52 wt.%, and the magnesium concentration was 0.8 wt.%.

### <Salt Substitution Step>

To 417g of the magnesium succinate solution obtained by the removal of bacterial cells, 16.3g ammonium carbonate was introduced, and allowed to react with stirring. As a result of continuing reaction for 2 hours, the solution became slurry state.

### <Solid-Liquid Separation Step>

The slurry reaction solution obtained above was centrifuged to separate precipitates, and an ammonium succinate solution was obtained. The succinic acid concentration of the solution was 4.52 wt.%. The molar ratio of ammonia and succinic acid was 1.57. The solution was dried, and the rate of succinic acid occupied in the total solid matter was measured and found to be 54.0%. The rate of succinic acid + ammonia occupied in the total solid matter was 66.2%.

The fermentation results in Example 1, Comparative Example 1 and Comparative Example 2 were compared in Table 6. It can be seen that good fermentation results can be obtained by neutralization with calcium.

**[Table 6]**

| | Example 1 | Comparative Example 1 | Comparative Example 2 |
|---|---|---|---|
| Succinic Acid (g/l) | 57.0 | 32.2 | 41.4 |
| Culture Time (hr) | 24 | 48 | 48 |
| Conversion Yield of Glucose (wt%) | 72 | 33 | 56 |

The analyzed results of the ammonium salt solutions obtained in Example 1, Comparative Example 1 and Comparative Example 2 are summarized in Table 7. It can be seen that the purity of the ammonium succinate solution produced by the calcium salt fermentation through the salt substitution is very high.

**[Table 7]**

| | Example 1 | Comparative Example 1 | Comparative Example 2 |
|---|---|---|---|
| Succinic Acid (wt%) | 10.1 | 3.17 | 4.52 |
| Acetic Acid (wt%) | 0.23 | 0.82 | 0.84 |
| NH₃ Conc. (wt%) | 2.83 | 0.95 | 1.02 |
| Ca Conc. (wt%) | 0.44 | 0.00 | 0.00 |
| Mg Conc. (wt%) | 0.00 | 0.00 | 0.80 |
| NH₃/Succinic Acid (mol/mol) | 1.92 | 2.08 | 1.57 |
| Succinic Acid/Total Solid Matter (wt%) | 72.7 | 44.2 | 54.0 |
| Succinic acid + NH₃/Total Solid Matter (wt%) | 93.1 | 57.5 | 66.2 |

### Industrial Applicability

By conducting the invention, it is made possible to produce ammonium succinate solutions efficiently without passing complicated processes, and from the ammonium succinate solution, succinic acid can be manufactured by a known method, and only reusable by-products for the manufacture of succinic acid are produced.

## Claims

1. A method of manufacturing an ammonium succinate solution with a high purity from fermentation comprising;
(1) crystallization fermentation step wherein calcium succinate trihydrate is produced by microorganism;
(2) transition step wherein calcium succinate trihydrate is converted to calcium succinate monohydrate by transition crystallization;
(3) crystal separation step wherein calcium succinate monohydrate is separated;
(4) salt substitution step wherein the calcium succinate monohydrate separated is made into ammonium succinate solution;
(5) solid-liquid separation step wherein calcium carbonate is removed from the ammonium succinate solution.

2. The method of manufacturing the ammonium succinate solution as set forth in claim 1 wherein calcium compound used in the fermentation is one or more selected from a group consisting of calcium hydroxide, calcium oxide, calcium hydrogen carbonate, and calcium carbonate.

3. The method of manufacturing the ammonium succinate solution as set forth in claim 1 wherein reaction to convert calcium succinate trihydrate to calcium succinate monohydrate is carried out at 80°C or more.

4. The method of manufacturing the ammoniun succinate solution as set forth in claim 1 wherein the salt substitution step to substitute ammonium succinate for calcium succinate monohydrate is carried out by ammonium carbonate or ammonia and carbon dioxide gas.

5. The method of manufacturing the ammonium succinate solution as set forth in claims 1-4 wherein succinic acid in the salt substitution step has a concentration of 5% by weight or more.

6. A method of manufacturing succinic acid comprising producing an ammonium succinate solution according to the method as set forth in either one of claims 1-5 and obtaining succinic acid from said ammonium succinate solution.

## Patentansprüche

1. Verfahren zum Herstellen einer Ammoniumsuccinatlösung mit einer hohen Reinheit durch Fermentation, welches umfasst:
(1) eine Kristallisationsfermentationsstufe, in der Calciumsuccinattrihydrat durch einen Mikroorganismus produziert wird;
(2) eine Übergangsstufe, in der Calciumsuccinattrihydrat durch Übergangskristallisation zu Calciumsuccinatmonohydrat umgewandelt wird;
(3) eine Kristallisationstrennstufe, in der Calciumsuccinatmonohydrat abgetrennt wird;
(4) eine Salzaustauschstufe, in der das abgetrennte Calciumsuccinatmonohydrat in eine Ammoniumsuccinatlösung überführt wird;
(5) eine Festflüssigtrennstufe, in der Calciumcarbonat aus der Ammoniumsuccinatlösung entfernt wird.

2. Verfahren zum Herstellen der Ammoniumsuccinatlösung nach Anspruch 1, wobei die in der Fermentation verwendete Calciumverbindung eine oder mehrere ist, die aus der aus Calciumhydroxid, Calciumoxid, Calciumhydrogencarbonat und Calciumcarbonat bestehenden Gruppe ausgewählt ist.

3. Verfahren zum Herstellen der Ammoniumsuccinatlösung nach Anspruch 1, wobei die Reaktion zur Umwandlung von Calciumsuccinattrihydrat zu Calciumsuccinatmonohydrat bei 80°C oder höher durchgeführt wird.

4. Verfahren zum Herstellen der Ammoniumsuccinatlösung nach Anspruch 1, wobei die Salzaustauschstufe zum Austauschen von Ammoniumsuccinat gegen Calciumsuccinatmonohydrat durch Ammoniumcarbonat oder Ammoniak und Kohlendioxidgas durchgeführt wird.

5. Verfahren zum Herstellen der Ammoniumsuccinatlösung nach einem der Ansprüche 1 bis 4, wobei Bernsteinsäure in der Salzaustauschstufe eine Konzentration von 5 Gew.-% oder mehr hat.

6. Verfahren zum Herstellen von Bernsteinsäure, welches das Herstellen einer Ammoniumsuccinatlösung nach dem Verfahren gemäß einem der Ansprüche 1 bis 5 und das Gewinnen von Bernsteinsäure aus der Ammoniumsuccinatlösung umfasst.

## Revendications

1. Procédé de fabrication d'une solution de succinate d'ammonium ayant une grande pureté par fermentation comprenant :
(1) une étape de fermentation avec cristallisation où du succinate de calcium trihydraté est produit par un microorganisme ;
(2) une étape de transition où le succinate de calcium trihydraté est converti en succinate de calcium monohydraté par cristallisation avec transition ;
(3) une étape de séparation des cristaux où le succinate de calcium monohydraté est séparé ;
(4) une étape de substitution de sel où le succinate de calcium monohydraté séparé est mis sous forme d'une solution de succinate d'ammonium ;
(5) une étape de séparation solide-liquide où du carbonate de calcium est retiré de la solution de succinate d'ammonium.

2. Procédé de fabrication de la solution de succinate d'ammonium selon la revendication 1 où un composé du calcium utilisé dans la fermentation est un ou plusieurs choisis dans un groupe consistant en l'hydroxyde de calcium, l'oxyde de calcium, l'hydrogénocarbonate de calcium et le carbonate de calcium.

3. Procédé de fabrication de la solution de succinate d'ammonium selon la revendication 1 où la réaction pour convertir le succinate de calcium trihydraté en succinate de calcium monohydraté est conduite à 80°C ou plus.

4. Procédé de fabrication de la solution de succinate d'ammonium selon la revendication 1 où l'étape de substitution de sel pour substituer le succinate d'ammonium au succinate de calcium monohydraté est accomplie par le carbonate d'ammonium ou l'ammoniac et le gaz dioxyde de carbone.

5. Procédé de fabrication de la solution de succinate d'ammonium selon les revendications 1-4 où l'acide succinique dans l'étape de substitution de sel a une concentration de 5 % en poids ou plus.

6. Procédé de fabrication d'acide succinique comprenant la production d'une solution de succinate d'ammonium selon le procédé présenté dans l'une quelconque des revendications 1-5 et l'obtention d'acide succinique à partir de ladite solution de succinate d'ammonium.
